# EUROPÄISCHE PATENTSCHRIFT

(11) **EP 0 292 885 B1**
(45) Veröffentlichungstag und Bekanntmachung des Hinweises auf die Patenterteilung: **08.09.1993**
(21) Anmeldenummer: 88108079.0
(22) Anmeldetag: 20.05.1988
(51) Int. Cl.: C07B 33/00, C07C 15/24, C07C 15/46, C07C 47/54, C07D 215/18, C07D 231/12, C07C 5/50, C07C 45/29

(54) **Katalytische Transferhydrierungen mit aromatischen Nitroverbindungen als Wasserstoffacceptor**
Catalytic transfer-hydrogenation with aromatic nitro compounds as hydrogen acceptor
Hydrogénation par transfert catalytique utilisant les composés nitro-aromatiques comme accepteur d'hydrogène

(30) Priorität: 27.05.1987 DE 3717881
(43) Veröffentlichungstag der Anmeldung: 30.11.1988
(73) Patentinhaber: BASF Aktiengesellschaft, 67063 Ludwigshafen (DE)
(72) Erfinder: Dockner, Toni, Dr., D-6701 Meckenheim (DE); Krug, Herbert, D-6700 Ludwigshafen (DE); Sauerwald, Manfred, Dr., D-6701 Roedersheim-Gronau (DE)

(56) Entgegenhaltungen:
- DE-A- 2 715 785
- GB-A- 2 052 294
- HOUBEN-WEYL: "Methoden der Organischen Chemie", Band IV/1b, Oxidation Teil 2, 1975, Seiten 967-982, Georg Thieme Verlag, Stutthart, DE

## Beschreibung

Die vorliegende Erfindung betrifft katalytische Transferhydrierungen mit aromatischen Nitroverbindungen als Wasserstoffacceptor und aktivierten organischen Verbindungen, bei denen durch Dehydrierung CC-, CO- oder CN-Doppelbindungen entstehen, als Wasserstoffdonor.

Die Dehydrierung aktivierter organischer Verbindungen mittels Nitroaromaten ist allgemein bekannt, z.B. aus Chemical Reviews, 74, Seiten 567 bis 580 (1974), Houben-Weyl, Methoden der Organischen Chemie Bd. IV/1b, Seiten 967 bis 982 (1976) und der DE-OS 27 15 785. Als Katalysatoren dienen z.B. Übergangsmetalle der Gruppe VIII des Periodensystems, insbesondere Edelmetalle wie Palladium oder Platin. Bei besonders aktivierten Ausgangsstoffen, die z.B. zu aromatischen Verbindungen dehydriert werden, wird die Umsetzung häufig in Abwesenheit von Katalysatoren durchgeführt, jedoch sind Umsätze und Selektivitäten nicht immer befriedigend.

Der Erfindung lag nun die Aufgabe zugrunde, ein Verfahren zur Dehydrierung organischer Verbindungen unter Ausbildung von CC-, CO- und CN-Doppelbindungen mittels Nitroaromaten zu finden, bei dem auf die Verwendung teurer Edelmetalle als Katalysatoren verzichtet werden kann und das insbesondere hohe Selektivitäten bei guten Umsätzen aufweist.

Demgemäß wurde gefunden, daß man katalytische Transferhydrierungen mit aromatischen Nitroverbindungen als Wasserstoffacceptor und organischen Verbindungen, bei denen durch Dehydrierung CC-, CO- oder CN-Doppelbindungen entstehen, als Wasserstoffdonor vorteilhaft durchführt, indem man Aktivkohle als Katalysator und gegebenenfalls eine Eisenverbindung als Cokatalysator verwendet und die Transferhydrierung bei Temperaturen von 50 bis 500°C vornimmt.

Als aromatische Nitroverbindungen können Nitrobenzol oder Nitroderivate anellierter Ringe wie Nitronaphthalin eingesetzt werden. Die Aromaten können ein oder mehrere, insbesondere 1 bis 3 inerte Substituenten tragen. Geeignete Substituenten sind u.a. Alkyl-, Alkenyl-, Aryl-, Aralkyl-, Amino-, Cyano-, Halogen-, Halogenalkyl, Hydroxi, Alkoxi, Carboxyl oder Sulfogruppen. Es können auch Nitrochinone wie z.B. Nitroanthrachinon oder Nitroderivate von heteroaromatischen Verbindungen wie Pyrrol, Thiophen, Benzothiophen, Chinolin, Isochinolin, Pyridin oder Imidazol verwendet werden. Diese können ebenso wie die benzoiden Systeme inerte Substituenten tragen. Die Nitroaromaten können außerdem weitere Nitrogruppen enthalten.

Beispielsweise seien folgende Verbindungen aufgeführt: Nitrobenzol, 4-Chlornitrobenzol, 1,3-Dinitrobenzol, 1-Chlor-2,6-dinitrobenzol, 3-Chlor-4-fluornitrobenzol, 2,4-Dimethoxynitrobenzol, 4-Cyanonitrobenzol, 3- oder 4-Nitrotoluol, 2,6-Dinitrotoluol, 2-Nitro-4-chlortoluol, 2-Nitro-6-chlortoluol, 4-Nitrophenol, o-Nitroanisol, m-Nitroanilin, 2,6-Dinitroanilin, 3-Nitrobenzoesäure, 3-Nitrobenzolsulfonsäure, Natrium-2-methyl-5-nitrobenzolsulfonat, 1-Nitronaphthalin, Nitroanthrachinon, Nitrochinolin, 2-Nitropyrrol, 2-Nitropyridin, 4(5)-Nitro-2-methylimidazol, 5-Nitro-2-trifluormethylbenzimidazol, 2-Nitrothiophen oder 3-Methyl-5-nitrobenzo(b)thiophen. Besonders bevorzugt ist Nitrobenzol.

Als Wasserstoffdonor geeignete organische Ausgangsstoffe sind Verbindungen, in denen Wasserstoffatome an benachbarten CC-, CO- oder CN-Einfachbindungen aktiviert sind, z.B. dadurch, daß durch die Dehydrierung ein stabilisiertes aromatisches System, das auch Heteroatome wie Stickstoff, Sauerstoff oder Schwefel enthalten kann, entsteht. Eine Aktivierung kann auch durch Phenylreste oder durch CC- oder CO-Doppelbindungen bewirkt werden, wenn die entstehende Doppelbindung in Konjugation mit dem Phenylrest oder der Doppelbindung treten kann.

Beispielsweise seien folgende Verbindungen aufgeführt: Tetralin, Δ2-Pyrazolin, 1,2,3,4-Tetrahydrochinolin, 1,2,3,4-Tetrahydroanthracen, 7-Chlor-8-methyl-1,2,3,4-tetrahydrochinolin, 2,3-Dihydrothiophen, Cyclohexen, Limonen, p-Menthen, 5-Isopropyl-2-methyl-1,3-cyclohexadien, 3-Phenylpropionaldehyd, Dehydrozimtsäure, Ethylbenzol, Isopropylbenzol oder Alkohol wie Benzylalkohol oder Isopropanol.

Die genannten Verbindungen können auch unter den Reaktionsbedingungen inerte Substituenten, z.B. C₁-C₄-Alkyl- oder Alkoxireste, Cyano-, Amino-, Halogen- oder Halogenalkylreste enthalten.

Erfindungsgemäß wird die Transferhydrierung in Gegenwart von Aktivkohle als Katalysator durchgeführt. Für das Verfahren geeignete Aktivkohle ist z.B. Tierkohle, die z.B. mit ZnCl₂, Phosphorsäure oder Wasserstoff aktiviert ist. Besonders vorteilhaft sind Aktivkohlen wie z.B. Carboraffin P® oder für Reaktionen in der Gasphase z.B. Supersorbon® oder Contarbon®. Die Katalysatorkohlen können grob-, mittel- und feinporig sein. Ihre Korngrößen betragen im allgemeinen 10 µm bis 4 mm, ihre spezifische innere Oberfläche ca. 5-15·10⁶ cm²/g (ermittelt nach Brunauer, Emmet, Teller, s. Ullmanns Encyklopädie d. techn. Chemie, 3. Aufl., Bd. 9, Seite 801 bis 812, 1957). Die Kohle kann pulverförmig oder in Formlingen eingesetzt werden.

Die Menge des Katalysators beträgt zweckmäßigerweise 0,5 bis 30 Gew.%, bezogen auf das gesamte Reaktionsgemisch. Größere Mengen sind möglich, bringen aber keine weiteren Vorteile.

Gemäß einer weiteren, vorteilhaften Ausführungsform des Verfahrens kann die Transferhydrierung in Gegenwart einer Eisenverbindung als Cokatalysator durchgeführt werden. Als Eisenverbindungen kommen zwei- und/oder dreiwertige Verbindungen in Betracht, z.B. Eisenhalogenide wie Chloride oder Bromide, Eisensulfat, Eisennitrat, Eisenphosphat, Eisenrhodanid, Eisenchromat, Eisen(II)-oxalat, Eisen(III)-acetat, Eisen(III)-formiat und insbesondere Eisenoxide, Eisenoxidhydrate oder Eisensulfide. Beispielsweise seien folgende Verbindungen aufgeführt: Eisen(II)-oxid, Eisen(III)-oxid (α- oder γ-Modifikation), Eisen(II,III)-oxid, Eisenoxidhydrat wie Goethit oder Lepidokrokit, Eisen(II)-sulfid, Eisen(II)-disulfid (Pyrit) oder Eisen(III)-sulfid. Es können auch Gemische der genannten Verbindungen verwendet werden. Besonders bevorzugt sind Eisen(II)-sulfid und -disulfid sowie insbesondere Fe₂O₃. Es ist auch möglich, Eisenpulver zuzusetzen.

Die Menge des Eisencokatalysators im Reaktionsgemisch kann ca. 0,01 bis 10 Gew.%, insbesondere 0,1 bis 5 Gew.%, bezogen auf den Wasserstoffacceptor, betragen. Man kann die Eisenverbindung dem Reaktionsgemisch z.B. in fester Form zusetzen, es ist aber auch möglich, Kohlepulver oder -formkörper vorher mit den Eisenverbindungen zu tränken, ggf. zu calcinieren, und dann die mit den Eisenverbindungen beladenen Aktivkohle-Kontakte einzusetzen, was sich insbesondere bei Reaktionen in der Gasphase anbietet. In solchen Fällen strebt man eine Beladung von 0,5 bis 30 Gew.% an Eisenverbindung, bezogen auf die gesamte Katalysatormenge, an.

Die Transferhydrierung kann in der Gasphase oder vorteilhaft in der Flüssigphase bei Temperaturen von 50 bis 500°C, insbesondere 100 bis 300°C, bei Über- oder Unterdruck oder vorteilhaft bei Normaldruck nach den dafür üblichen Techniken durchgeführt werden.

Allgemein kann die Menge an eingesetztem Wasserstoffdonor in weiten Grenzen schwanken. Beispielsweise kann die organische Verbindung sowohl als Wasserstoffdonor als auch als Verdünnungsmedium für die Reaktion verwendet werden. In solch einem Fall liegt der Donor üblicherweise in großem Überschuß vor. Andererseits kann es in manchen Fällen wünschenswert sein, die stöchiometrisch erforderliche Menge oder nur einen geringen Überschuß an Donor zu verwenden. In einem solchen Fall kann das Reaktionsgemisch auch ein zusätzliches flüssiges Verdünnungsmittel (Lösungsmittel) enthalten.

Geeignete Lösungs- oder Verdünnungsmittel sind z.B. aromatische oder aliphatische Kohlenwasserstoffe wie Toluol, Benzol, o-, m-, p-Xylol, hochsiedende Kohlenwasserstoffe oder Kohlenwasserstoffgemische, Chlorkohlenwasserstoffe, Ether oder stickstoffhaltige Lösungsmittel wie tertiäre Amine, Pyridin, tertiäre Amide wie Dimethylformamid oder N-Methylpyrrolidon oder insbesondere die aromatische Aminoverbindung, die durch Reduktion als eingesetzten Nitroaromaten entsteht. Vorzugsweise wird die Transferhydrierung in Abwesenheit eines zusätzlichen Lösungsmittels durchgeführt.

Die Transferhydrierung läßt sich sehr leicht ausführen, indem man z.B. den Wasserstoffdonor zusammen mit dem Katalysator gegebenenfalls in Gegenwart eines Lösungsmittels auf Reaktionstemperatur erhitzt und die aromatische Nitroverbindung hinzufügt. Es ist auch möglich, den Wasserstoffacceptor vorzulegen und den Donor hinzuzumischen oder beide Komponenten zusammen in Gegenwart von Aktivkohle zu erhitzen. Sich bildendes Reaktionswasser kann während der Umsetzung z.B. durch Destillation entfernt werden.

Die Aufarbeitung des Reaktionsgemisches kann in an sich bekannter Weise erfolgen, indem man z.B. den Katalysator abfiltriert und die Produkte aus dem Filtrat destillativ oder durch Extraktion abtrennt.

Bei Umsetzungen in der Gasphase werden die Reaktionspartner zweckmäßigerweise in einem Vorverdampfer verdampft und gegebenfalls mittels eines Inertgasstromes, z.B. Stickstoffstromes in den, den Katalysator enthaltenden, Reaktor eingetragen. Die Aktivkohle liegt zweckmäßigerweise in Form von Tabletten oder Strängen oder als Wirbelkontakt mit Kohlenstoffpartikeln unterhalb von 1 mm vor.

Die den Reaktor verlassenden Gase werden kondensiert und die Produkte in an sich bekannter Weise aus dem anfallenden Kondensat isoliert.

### Beispiel 1

In einer Rührapparatur, versehen mit Tropftrichter und Rückflußkühler, werden 200 g Anilin, 50 g Nitrobenzol und 30 g Carboraffin P®, das 5 Gew.% Eisen(II)sulfid enthält, auf 130°C erhitzt. Dann tropft man innerhalb 2 Stunden 42 g Pyrazolin zu. Nach Abkühlen und Abfiltrieren erhält man 272 g Filtrat mit 1,4 Gew.% Pyrazolin und 8,9 Gew.% Pyrazol. Dies entspricht einem Umsatz von 91 % und einer Selektivität von 65 %.

### Beispiel 1a

Man verfährt wie in Beispiel 1, arbeitet aber ohne Carboraffin P® und Eisensulfid. Nach dem Abfiltrieren erhält man 271 g Filtrat mit 15,5 Gew.% Pyrazolin. Dies entspricht der eingesetzten Menge.

### Beispiel 2

40 g 7-Chlor-8-methyltetrahydrochinolin werden mit 60 g eines Kohlenwasserstoffgemisches mit einem Siedepunkt oberhalb von 350°C und 10 g Carboraffin P® unter Rühren auf 250°C erhitzt. Dann gibt man 22 g 6-Chlor-2-nitrotoluol dazu und hält 15 Minuten bei 250°C.

Nach dem Abkühlen auf Raumtemperatur wird abfiltriert und mit 100 g Toluol nachgewaschen. Man erhält 212 g Filtrat mit 2,3 Gew.% 7-Chlor-8-methyltetrahydrochinolin und 15,7 Gew.% 7-Chlor-8-methylchinolin. Dies entspricht einem Umsatz von 88 % und einer Selektivität von 97 %. Im Filtrat ist kein 6-Chlor-2-nitrotoluol mehr nachweisbar.

### Beispiel 2a

Man verfährt wie in Beispiel 2, arbeitet aber ohne Zusatz von Carboraffin P®.

Man erhält 213 g Filtrat mit 16 Gew.% 7-Chlor-8-methyltetrahydrochinolin und 2,65 Gew.% 7-Chlor-8-methylchinolin. Dies entspricht einem Umsatz von 15 % und einer Selektivität von 97,5 %.

### Beispiel 3

40 g Benzylalkohol, 10 g Nitrobenzol und 5 g getrocknetes Aktivkohlepulver Carboraffin P® werden in einem 100-ml-Glasautoklaven 2 Stunden bei 250°C gerührt. Nach dem Abkühlen auf Raumtemperatur wird die Reaktionsmischung abfiltriert und das Filtrat (49 g) gaschromatographisch analysiert.

Man erhält folgende Zusammensetzung:
55,0 % Benzylalkohol
23,7 % Benzaldehyd
9,0 % Nitrobenzol
7,1 % Anilin
Der Umsatz an Benzylalkohol zu Benzaldehyd ist 32,5 % und die Selektivität 92 %; der Umsatz an Nitrobenzol ist 56 %, aus Nitrobenzol bildet sich zu 82 % Anilin.

### Beispiel 3a

Bei einem unter den Bedingungen von Beispiel 3 durchgeführten Vergleichsversuch ohne Zusatz von Carboraffin P® erhält man folgende Zusammensetzung:
79,8 % Benzylalkohol
1,0 % Benzaldehyd
16,2 % Nitrobenzol
Rest Nebenprodukte
Der Umsatz an Benzylalkohol ist <2 %.

### Beispiel 4

In einer Rührapparatur mit Tropftrichter und Wasserauskreiser werden 99 g (≙ 0,75 mol) Tetralin und 16 g Carboraffin P® vorgelegt. Das Gemisch wird unter Rühren auf 250°C erhitzt. Dann werden innerhalb von 3h 61,5 g (≙ 0,5 mol) Nitrobenzol zugetropft. Das überdestillierte Produkt wird nach und nach wieder in den Kolben zurückgegeben. Das gebildete Wasser wird ausgekreist. Nach 10 Stunden wird aus dem Reaktionsgemisch eine Probe entnommen und abfiltriert.

Man erhält:
4,15 % Anilin
28,5 % Nitrobenzol
55,9 % Tetralin
9,50 % Naphthalin
Dies entspricht einem Umsatz an Tetralin von 13,7 % und einer Selektivität von 99 %.

### Beispiel 4a

Man verfährt wie im Beispiel 4, jedoch ohne den Zusatz von Carboraffin P®.

Man erhält nach 10 Stunden:
0,39 % Anilin
35,6 % Nitrobenzol
57,8 % Tetralin
0,63 % Naphthalin
Dies entspricht einem Umsatz <2 %.

### Beispiel 5

Im Laufe von 5 Stunden wird ein Gemisch von 165 g Ethylbenzol und 55,1 g Nitrobenzol bei 275°C in einem Flashverdampfer verdampft und mit 3 l Stickstoff/Stunde in den Reaktor eingetragen, der aus einem beheizten zylindrischen Quarzrohr von 50 cm Länge besteht und 250 ml Aktivkohlegranulat Supersorbon® in 2 mm Strängen enthält. Die Temperatur des Reaktors ist 350°C.

Nach 5 Stunden haben sich in Vorlage und Kühlfalle 209,5 g Kondensat gesammelt, das aus 10,5 g Wasser und 199 g organischer Phase besteht. Die gaschromatographische Analyse der organischen Phase ergibt:
144,1 g Ethylbenzol
16,1 g Styrol
8,2 g Anilin
29,5 g Nitrobenzol
1,1 g nicht idendifiziertes Nebenprodukt
Danach beträgt der Umsatz von Ethylbenzol zu Styrol 12,9 % und die Selektivität 77 %.

Der Umsatz von Nitrobenzol zu Anilin beträgt 46,4 %, die Selektivität 42,3 %.

## Patentansprüche

1. Katalytische Transferhydrierungen mit aromatischen Nitroverbindungen als Wasserstoffacceptor und organischen Verbindungen, bei denen durch Dehydrierung CC-, CO- oder CN-Doppelbindungen entstehen, als Wasserstoffdonor, dadurch gekennzeichnet, daß man Aktivkohle als Katalysator und gegebenenfalls einen eisenhaltigen Cokatalysator verwendet und die Transferhydrierung bei Temperaturen von 50 bis 500°C vornimmt.

2. Verfahren nach Anspruch 1, dadurch gekennzeichnet, daß man Nitrobenzol oder substituiertes Nitrobenzol als Wasserstoffacceptor verwendet.

3. Verfahren nach Anspruch 1, dadurch gekennzeichnet, daß man Nitronaphthalin oder Nitroanthrachinon als Wasserstoffacceptor verwendet.

4. Verfahren nach den Ansprüchen 1 bis 3, dadurch gekennzeichnet, daß man als Wasserstoffdonor Di- oder Tetrahydroaromaten verwendet.

5. Verfahren nach den Ansprüchen 1 bis 3, dadurch gekennzeichnet, daß man als Wasserstoffdonor durch Phenylreste oder durch CC- oder CO-Doppelbindungen aktivierte organische Verbindungen verwendet.

6. Verfahren nach den Ansprüchen 1 bis 6, dadurch gekennzeichnet, daß man als Cokatalysator Eisenoxid, Eisenoxidhydrat, Eisensulfid oder Gemische hiervon verwendet.

## Claims

1. A catalytic transfer hydrogenation using an aromatic nitro compound as the hydrogen acceptor and an organic compound which on dehydrogenation gives rise to CC, CO or CN double bonds as the hydrogen donor, which comprises using activated carbon as the catalyst with or without an iron-containing cocatalyst and carrying out the transfer hydrogenation at from 50 to 500°C.

2. A process as claimed in claim 1, wherein the hydrogen acceptor used is nitrobenzene or substituted nitrobenzene.

3. A process as claimed in claim 1, wherein the hydrogen acceptor used is nitronaphthalene or nitroanthraquinone.

4. A process as claimed in any of claims 1 to 3, wherein the hydrogen donor used is a di- or tetrahydroaromatic.

5. A process as claimed in any of claims 1 to 3, wherein the hydrogen donor used is an organic compound activated by phenyl or CC or CO double bonds.

6. A process as claimed in any of claims 1 to 6, wherein the cocatalyst used is iron oxide, hydrated iron oxide, iron sulfide or a mixture thereof.

## Revendications

1. Hydrogénation catalytique par transfert avec, comme accepteurs d'hydrogène, des composés aromatiques nitrés et, comme donneurs d'hydrogène, des composés organiques dans lesquels des doubles liaisons CC, CO ou CN sont formées par déshydrogénation, caractérisée en ce qu'on utilise du charbon actif comme catalyseur et éventuellement un co-catalyseur contenant du fer, et en ce qu'on procède à l'hydrogénation par transfert à des températures de 50 à 500°C.

2. Procédé selon la revendication 1, caractérisé en ce qu'on utilise, comme accepteur d'hydrogène, du nitrobenzène ou un nitrobenzène substitué.

3. Procédé selon la revendication 1, caractérisé en ce qu'on utilise, comme accepteur d'hydrogène, un nitronaphtalène ou une nitroanthraquinone substituée.

4. Procédé selon l'une quelconque des revendications 1 à 3, caractérisé en ce qu'on utilise, comme donneurs d'hydrogène, des carbures di- ou tétrahydroaromatiques.

5. Procédé selon l'une quelconque des revendications 1 à 3, caractérisé en ce qu'on utilise, comme donneurs d'hydrogène, des composés organiques activés par des restes phényle ou par des doubles liaisons CC ou CO.

6. Procédé selon l'une quelconque des revendications 1 à 5, caractérisé en ce qu'on utilise, comme cocatalyseur, de l'oxyde de fer, de l'oxyde de fer hydraté, du sulfure de fer ou des mélanges de ceux-ci.
